(19) Europäisches Patentamt
European Patent Office
Office européen des brevets

(11) Veröffentlichungsnummer: 0 350 688 B1

(12) **EUROPÄISCHE PATENTSCHRIFT**

(45) Veröffentlichungstag der Patentschrift: **14.04.93**

(51) Int. Cl.⁵: **C07C 233/61**, A01N 53/00

(21) Anmeldenummer: **89111533.9**

(22) Anmeldetag: **24.06.89**

# (54) Cyclopropancarboxamide, Verfahren zu ihrer Herstellung und ihre Verwendung zur Bekämpfung von Schädlingen.

(30) Priorität: **12.07.88 DE 3823521**

(43) Veröffentlichungstag der Anmeldung:
**17.01.90 Patentblatt 90/03**

(45) Bekanntmachung des Hinweises auf die Patenterteilung:
**14.04.93 Patentblatt 93/15**

(84) Benannte Vertragsstaaten:
**BE CH DE ES FR GB IT LI NL**

(56) Entgegenhaltungen:
**EP-A- 0 258 733**
**DE-A- 2 611 695**

(73) Patentinhaber: **BASF Aktiengesellschaft**
**Carl-Bosch-Strasse 38**
**W-6700 Ludwigshafen(DE)**

(72) Erfinder: **Kardorff, Uwe, Dr.**
**D 3,4**
**W-6800 Mannheim 1(DE)**
Erfinder: **Neubauer, Hans-Jürgen, Dr.**
**Mozartstrasse 6**
**W-4400 Münster-Hiltrup(DE)**
Erfinder: **Leyendecker, Joachim, Dr.**
**Stahlbühlring 79**
**W-6802 Ladenburg(DE)**
Erfinder: **Künast, Christoph, Dr.**
**Salierstrasse 2**
**W-6701 Otterstadt(DE)**
Erfinder: **Hofmeister, Peter, Dr.**
**Bernard-Humblot-Strasse 12**
**W-6730 Neustadt(DE)**
Erfinder: **Krieg, Wolfgang, Dr.**
**Saarstrasse 17**
**W-6721 Weingarten(DE)**

EP 0 350 688 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Cyclopropancarboxamide der allgemeinen Formel I

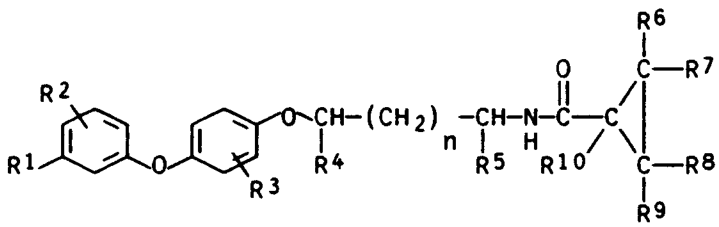
(I)

in der n für 0 oder 1 steht und die Substituenten folgende Bedeutung haben:

- $R^1$ Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, Nitro oder Cyano
- $R^2$ Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, Nitro oder Cyano
- $R^3$ Wasserstoff, Halogen oder $C_1$-$C_3$-Alkyl
- $R^4$, $R^5$ Wasserstoff oder $C_1$-$C_4$-Alkyl und
- $R^6$-$R^{10}$ Wasserstoff, Halogen oder $C_1$-$C_4$-Alkyl

mit der Maßgabe, daß die Reste $R^4$-$R^{10}$ nicht alle gleichzeitig für Wasserstoff stehen.

Außerdem betrifft die vorliegende Erfindung die Herstellung der Verbindung I, Schädlingsbekämpfungsmittel, die die Verbindung I enthalten und ein Verfahren zur Bekämpfung von Schädlingen.

Aus der DE-A 26 33 069 ist der Cyclopropancarbonsäureester A

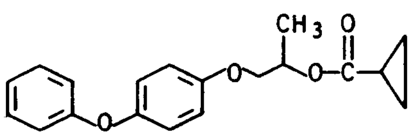
(A)

und aus der DE OS 36 28 082 Cyclopropancarbonsäureamide der Struktur B

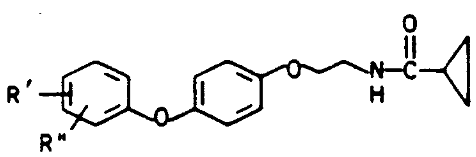
(B) ,

worin R' und R" Wasserstoff, Halogen, $C_1$-$C_3$-Halogenalkoxy oder Methylthio bedeuten, als Schädlingsbekämpfungsmittel bekannt.

Die Wirkung und Freilandstabilität der Verbindungen A und B lassen jedoch zu wünschen übrig.

Der Erfindung lag nun die Aufgabe zugrunde, neue Cyclopropancarboxamide I mit verbesserter Wirkung und/oder gegen andere Schädlinge wirksamere Verbindungen I bereitzustellen.

Demgemäß wurden die eingangs definierten neuen Carboxamide I sowie Verfahren zu deren Herstellung gefunden. Ferner wurde gefunden, daß sich die Verbindungen I hervorragend zur Bekämpfung von Schädlingen eignen.

Die Cyclopropancarboxamide I sind aus den 4-Phenoxyphenoxy-alkylaminen II

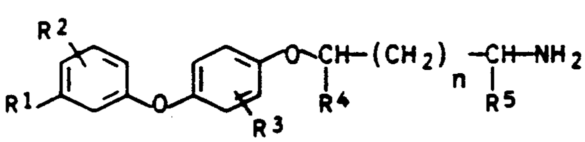
(II)

durch Umsetzung mit den entsprechenden Cyclopropancarbonsäurehalogeniden III

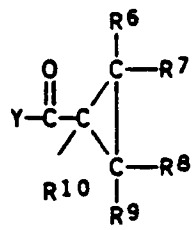

(III),

worin Y für Halogen wie Fluor, Chlor, Brom oder Jod, vorzugsweise Brom oder Chlor, insbesondere Chlor steht, in Gegenwart eines Säureakzeptors gut zugänglich.

Als Säureakzeptor kann das 4-Phenoxyphenoxy-alkylamin II eingesetzt werden, üblicherweise verwendet man jedoch an sich übliche Basen als säurebindendes Mittel, insbesondere aliphatische aromatische oder heterocyclische Amine, beispielsweise Triethylamin, Dimethylamin, Diisopropylamin, Piperidin, Dimethylanilin, Dimethylbenzylamin, Pyridin und 4-Dimethylaminopyridin; Hydroxide von Alkali- und Erdalkalimetallen wie zum Beispiel Natriumhydroxid, Kaliumhydroxid, Calciumhydroxid; Alkoholate von Alkali- und Erdalkalimetallen wie bespielsweise Natriummethylat, Natriumethylat, Calciummethylat, Kalium-tert.-butylat; Alkali- oder Erdalkalimetallhydride wie beispielsweise Natriumhydrid, Kaliumhydrid oder Calciumhydrid; Alkali- oder Erdalkalimetallcarbonate wie beispielsweise Natriumcarbonat, Kaliumcarbonat oder Calciumcarbonat.

Das Verhältnis Säureakzeptor zu Säurehalogenid III ist nicht besonders kritisch und liegt im allgemeinen bei 0,5 : 1 bis 20 : 1, vorzugsweise 0,7 : 1 bis 5 : 1, besonders bevorzugt 0,9 : 1 bis 1,5 : 1.

Üblicherweise setzt man die Ausgangsstoffe II und III in stöchiometrischem Verhältnis ein. Ein Überschuß der einen oder anderen Komponente kann in Einzelfällen aber durchaus vorteilhaft sein.

Die Umsetzung verläuft gewöhnlich oberhalb von -30 °C mit ausreichender Geschwindigkeit. Im allgemeinen liegt die Temperatur bei -30 bis 130 °, insbesondere -10 bis 80 °C, bevorzugt 0 bis 50 °C. Da die Umsetzung in einigen Fällen unter Wärmeentwicklung abläuft, kann es vorteilhaft sein, eine Kühlmöglichkeit vorzusehen.

Die Umsetzung wird zweckmäßigerweise in einem Lösungs- oder Verdünnungsmittel bei Normaldruck, im Über- oder Unterdruck durchgeführt.

Als Lösungs- oder Verdünnungsmittel eignen sich aliphatische, aromatische, gegebenenfalls chlorierte Kohlenwasserstoffe wie beispielsweise Petrolether, n-Pentan, n-Hexan, Hexan-Isomerengemische, Benzol, Toluol, Xylol, Benzin, Dichlormethan, Chloroform, Tetrachlorkohlenstoff, 1,2-Dichlorethan und Chlorbenzol; Ether und Ester wie Diethyl- und Di-n-butylether, Methyl-tert.-butylether, Tetrahydrofuran, Dioxan, Essigsäureethylester; Ketone wie Aceton, Methylethylketon, Methylisopropylketon, Nitrile wie Aceto- und Propionitril; Alkohole wie Methanol, Ethanol, n-Propanol, Isopropanol, aprotische dipolare Lösungsmittel wie Dimethylformamid, Dimethylsulfoxid, Pyridin. Auch Gemische dieser Stoffe können als Lösungs- und Verdünnungsmittel verwendet werden.

Die zur Herstellung der Verbindungen I benötigten 4-Phenoxyphenoxy-alkylamine II sind zum Teil aus Houben/Weyl, Bd. VI, 3, Methoden der organischen Chemie, Thieme Verlag, 1965, 85 ff, bekannt oder können nach den dort beschriebenen Methoden hergestellt werden.

Die außerdem benötigten Säurehalogenide III sind bekannt und insbesondere die Säurechloride größtenteils kommerziell erhältlich.

Die erfindungsgemäßen Verbindungen der Formel I können außerdem noch nach praktisch allen bekannten Verfahren der Carboxamidsynthese dargestellt werden, beispielsweise durch Umsetzung von 4-Phenoxyphenoxy-alkylaminen II mit entsprechenden Cyclopropancarbonsäureestern, Cyclopropancarbonsäuren oder deren Salze, Cyclopropancarbonsäureanhydriden oder Ketenderivaten (vgl. C. Ferri, Reaktionen der organischen Synthese, Georg Thieme Verlag, Stuttgart, 1978, S. 542 und dort zitierte Literatur).

Die neuen Verbindungen der Formel I fallen teilweise in Form farbloser oder schwach bräunlich gefärbter Öle an, die sich durch längeres Erwärmen unter vermindertem Druck auf mäßig erhöhte Temperaturen ("Andestillieren") von den letzten flüchtigen Anteilen befreien und auf diese Weise reinigen lassen. Erhält man die Verbindungen der Formel I in kristalliner Form, so kann ihre Reinigung durch Umkristallisation erfolgen.

In den Verbindungen der Formel I haben die Reste $R^1$-$R^{10}$ im einzelnen die folgende Bedeutung:

$R^1$
- Wasserstoff
- Halogen wie Fluor, Chlor, Brom und Iod, bevorzugt Fluor, Chlor und Brom,
- unverzweigtes oder verzweigtes $C_1$-$C_4$-Alkyl wie Methyl, Ethyl, Propyl und Butyl, bevorzugt für Methyl oder Ethyl für verzweigtes Alkyl wie Isopropyl, Isobutyl und sec.Butyl,
- unverzweigtes oder verzweigtes $C_1$-$C_4$-Halogenalkyl, bevorzugt Fluor- und Chloralkyl wie Fluormethyl, Difluormethyl, Trifluormethyl, Pentafluorethyl, 2,2,2-Trifluorethyl, Trichlormethyl und 2,2,2-Trichlorethyl,
- unverzweigtes oder verzweigtes $C_1$-$C_4$-Alkoxy, bevorzugt Methoxy, Ethoxy und Isopropoxy,
- unverzweigtes oder verzweigtes $C_1$-$C_4$-Halogenalkoxy, bevorzugt Fluor- und Chloralkoxy wie Trifluormethoxy, 1,1,2,2-Tetrafluorethoxy, Pentafluorethoxy, Trichlormethoxy.
- Nitro, Cyano;

$R^2$
- Wasserstoff,
- Halogen wie Fluor, Chlor, Brom und Iod, bevorzugt Fluor, Chlor und Brom,
- unverzweigtes oder verzweigtes $C_1$-$C_4$-Alkyl wie Methyl, Ethyl, Propyl und Butyl, bevorzugt für Methyl oder Ethyl und für verzweigtes Alykl wie Isopropyl, Isobutyl und sec.Butyl,
- unverzweigtes oder verzweigtes $C_1$-$C_4$-Halogenalkyl, bevorzugt Fluor- und Chloralkyl wie Fluormethyl, Difluormethyl, Trifluormethyl, Pentafluorethyl, 2,2,2-Trifluorethyl, Trichlormethyl und 2,2,2-Trichlorethyl,
- unverzweigtes oder verzweigtes $C_1$-$C_4$-Alkoxy bevorzugt Methoxy, Ethoxy und Isopropoxy,
- unverzweigtes oder verzweigtes $C_1$-$C_4$-Halogenalkoxy, bevorzugt Fluor- und Chloralkoxy wie Trifluormethoxy, 1,1,2,2-Tetrafluorethoxy, Pentafluorethoxy, Trichlormethoxy,
- Nitro, Cyano;

$R^3$
- Wasserstoff,
- Halogen wie Fluor, Chlor und Brom, bevorzugt Fluor und Chlor,
- unverzweigtes oder verzweigtes $C_1$-$C_3$-Alkyl wie Methyl, Ethyl, Propyl, Isopropyl,

$R^4$, $R^5$
- Wasserstoff,
- Halogen wie Fluor, Chlor und Brom, bevorzugt Fluor und Chlor,
- unverzweigtes oder verzweigtes $C_1$-$C_4$-Alkyl wie Methyl, Ethyl, Propyl, Butyl, bevorzugt für Methyl oder Ethyl, und für verzweigtes Alkyl wie Isopropyl, Isobutyl und sec.Butyl;

$R^6$-$R^{10}$
- Wasserstoff,
- Halogen wie Fluor, Chlor und Brom, bevorzugt Fluor und Chlor,
- unverzweigtes oder verzweigtes $C_1$-$C_4$-Alkyl wie Methyl, Ethyl, Propyl, Butyl, bevorzugt Methyl, Ethyl und Isopropyl;

mit der Maßgabe, daß die Reste $R^4$-$R^{10}$ nicht alle gleichzeitig für Wasserstoff stehen.

Besonders bevorzugt sind Verbindungen I, in denen $R^2$ und $R^3$ Wasserstoff bedeuten.

Die erfindungsgemäßen Verbindungen I können ein oder mehrere Asymmetriezentren enthalten. Die vorliegende Erfindung schließt alle möglichen Stereoisomeren wie Diastereomere, Enantiomere sowie Diastereomeren- und Enantiomerengemische ein.

Im Gegensatz zu den meisten bisher bekannten Schädlingsbekämpfungsmitteln, die als Kontakt- oder Fraßgifte die Tiere töten, lähmen oder vertreiben, greifen die Verbindungen der Formel I in das Hormonsystem des tierischen Organismus ein. Bei Insekten wird beispielsweise die Umwandlung zur Imago, die Ablage von entwicklungsfähigen Eiern und die Entwicklung von abgelegten, normalen Eiern gestört und dadurch die Generationsfolge unterbrochen. Für Wirbeltiere sind die erfindungsgemäßen Mittel praktisch ungiftig.

Die meisten Verbindungen der Formel I werden überdies leicht zu Stoffen abgebaut, die in der natürlichen Umgebung vorkommen und von Mikroorganismen weiter zerlegt werden.

Die Cyclopropancarboxamide der allgemeinen Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellonella (Wachsmotte), Malacosoma neustria (Ringelspinner), Dendrolimus pini (Kiefernspinner), Thaumatopoea ptiyocampa (Pinienprozessionsspinner), Phalera bucephala (Mondfleck), Cheimatobia brumata (Kleiner Frostspanner), Hibernia defoliaria (Großer Frostspanner), Bupalus piniarius (Klefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling);

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varivestis (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagai (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-puncta (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Ortiorrhynchus sulcatus (Gefurchter Lappenrüßler), Otiorrhynchus ovatus (Erdbeerwurzelrüßler), Hylobius abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner);

aus der Ordnung der Zweiflügler (Diptera) beispielsweise Lycoria pectoralis, Mayetiola destructor (Hessenfliege), Basineura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hysocyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ovis, Chrysomya macellaria, Chrysomya hominivorax, Lucilia cuprina, Lucilia sericata, Hypoderma lineata;

aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuerameise), Atta sexdens (Blattschneiderameise);

aus der Ordnung der Wanzen (Heteroptera) beispielsweise Nezara viridula (Grüne Reiswanze), Eurygaster integriceps (Asiatische Getreidewande), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);

aus der Ordnung der Pflanzensauger (Homoptera) beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlauf), Aphidula nasturtii (Kreuzdornblattlaus), Cerosipha gossypii (Gurkenblattlauf), Sappaphis mali (Rosige Apfellaus), Sappaphis mala (Mehlige Birnblattlaus), Dysaphis radicola (Mehige Apfelfaltenlaus), Brachycaudus cardui (Große

Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenlaus), Macrosiphon rosae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);
aus der Ordnung der Termiten (Isoptera) beispielsweise Reticulitermes lucifugus, Calotermes flavicollis, Leucotermes flavipes, Termes natalensis;
aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melanoplus femur-rubrum (Rotbeinige Heuschrecke), Blatta orientalis (Küchenschabe), Blattella germanica (Deutsche Schabe), Periplaneta americana (Amerikanische Schabe), Blabera gigantes (Riesenschabe).

Zur Klasse der Arachnoidea gehören Spinnentiere (Acarina) beispielsweise Ixodes ricinus (Holzbock), Ornithodorus moubata, Amblyomma americanum, Dermacentor silvarum, Boophilus microplus, Tetranychus telarius, Tetranychus pacificus, Paratetranychus pilosus, Bryobia praetiosa.

Zur Klasse der Nematoden zählen beispielsweise Wurzelgallennematoden, z.B. Meloidogyne incognita, Meloidogyne hapla, Meloidogyne javanica, Zysten bildende Nematoden, z.B. Globodera rostochiensis, Heterodera schachtii, Heterodera avenae, Heterodera glycinae, Heterodera trifolii, Stock- und Blattälchen, z.B. Ditylenchus dipsaci, Ditylenchus destructor, Pratylenchus neglectus, Pratylenchus penetrans, Pratylenchus goodeyi, Paratylenchus curvitatus sowie Tylenchorhynchus dubius, Tylenchorhynchus claytoni, Rotylenchus robustus, Heliocotylenchus multicinctus, Radopholus similis, Belonolaimus longicaudatus, Longidorus elongatus, Trichodorus primitivus.

Die Wirkstoffe können als solche, in Form ihrer Formulierungen oder den daraus bereiteten Anwendungsmöglichkeiten, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkali, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Aklylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolylglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 5 Gew.-Teile der Verbindung Nr. 2 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr. 2 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr. 1 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 3 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. 12 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variiert werden.

Im allgemeinen liegen sie zwischen 0,0001 und 10 Gew.-%, vorzugsweise zwischen 0,01 und 1 Gew.-%. Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,02 bis 10, vorzugsweise 0,05 bis 2,0 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Herstellungsbeispiele:

Beispiel 1

N-[2-(4-Phenoxy-phenoxy)-prop-1-yl]-cyclopropancarboxamid

12,15 g 2-(4-Phenoxy-phenoxy)propylamin werden zusammen mit 10,1 g Triethylamin in 100 ml Diethylether vorgelegt und 5,2 g Cyclopropancarbonsäurechlorid, in 50 ml Diethylether gelöst, bei 25 °C dazugetropft. Nach Beendigung der leicht exothermen Reaktion wird 14 h bei 25 °C gerührt, das Lösungsmittel entfernt und der Rückstand in Essigsäureethylester aufgenommen. Es wird gründlich mit Wasser gewaschen, über Natriumsulfat getrocknet und im Vakuum eingeengt. Dadurch verbleibt ein Feststoff, der aus Hexan/Essigester umkristallisiert wird. Man erhält weiße Kristalle vom Festpunkt 74 °C.

Ausbeute: 13,2 g; 85 % der rechnerisch möglichen Menge.

| $C_{19}H_{21}NO_3$ (311,38) | | | |
|---|---|---|---|
| ber.:<br>gef.: | C: 73,29 %;<br>C: 73,2 %; | H: 6,80 %;<br>H: 6,8 %; | N: 4,5 %;<br>N: 4,4 %; |

Infrarotabsorptionen [$cm^{-1}$]: 1504, 1490, 1258, 1225, 1194, 1117, 757.

Beispiel 2

N{2-[4-(3-Fluorphenoxy)-phenoxy]prop-1-yl}-2-methylcyclopropancarboxamid

Zu 3,95 g [4-(3-Fluor)phenoxy-phenoxy]-propylamin in 17,5 ml Pyridin werden 1,9 g 2-Methylcyclopropancarbonsäurechlorid in 5,8 ml Dichlormethan bei Raumtemperatur zugetropft und nach Beendigung des Zutropfens 14 h bei Raumtemperatur gerührt. Dann wird in die vierfache Menge Wasser gegossen und die organische Phase abgetrennt. Die Wasserphase wird mit Dichlormethan extrahiert und die vereinigten organischen Phasen mit Wasser gewaschen. Nach Trocknen über Natriumsulfat wird im Rotationsverdampfer unter Vakuum eingeengt und der verbleibende Rückstand über eine Kieselgel-Flash-Säule mit Toluol-Essigester = 8:2 als Laufmittelgemisch chromatographiert. Nach Entfernen der Laufmittel erhält man weiße Kristalle, Festpunkt 60-62 °C.

Ausbeute: 4,4 g; 85 % rechnerisch möglichen Menge.

| $C_{20}H_{22}FNO_3$ (343,40) | | | |
|---|---|---|---|
| ber.:<br>gef.: | C: 69,95 %;<br>C: 70,3 %; | H: 6,46 %;<br>H: 6,7 %; | N: 4,08 %;<br>N: 4,1 %; |

Infrarotabsorption [$cm^{-1}$]: 1501, 1483, 1237, 1219, 1205, 1118, 960.

Entsprechend dieser Darstellungsbeispiele können die in der folgenden Tabelle beschriebenen Verbindungen I hergestellt werden. Im Falle nicht kristalliner Substanzen, werden die intensivsten Infrarotabsorptionen im Bereich kleiner ~ 1500 $cm^{-1}$ angegeben.

Tabelle 1: Cyclopropancarboxamide I

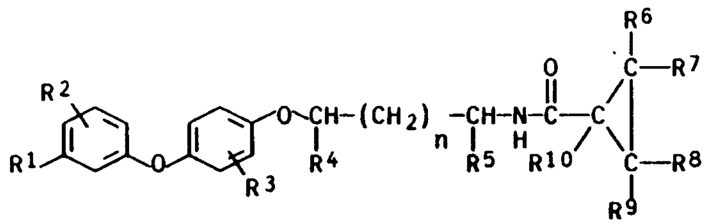

(I)

| Beispiel | n | $R^1$ | $R^2$ | $R^3$ | $R^4$ | $R^5$ | $R^6$ | $R^7$ | $R^8$ | $R^9$ | $R^{10}$ | Schmp. (°C) bzw. IR-Daten ($cm^{-1}$) |
|---|---|---|---|---|---|---|---|---|---|---|---|---|
| 1 | 0 | H | H | H | $CH_3$ | H | H | H | H | H | H | 74 |
| 2 | 0 | F | H | H | $CH_3$ | H | $CH_3$ | H | H | H | H | 60-62 |
| 3 | 0 | H | H | H | H | $CH_3$ | H | H | H | H | H | 101 |
| 4 | 0 | F | H | H | $CH_3$ | H | H | H | H | H | H | 85-86 |
| 5 | 0 | Cl | H | H | $CH_3$ | H | H | H | H | H | H | 74-75 |
| 6 | 0 | Br | H | H | $CH_3$ | H | H | H | H | H | H | 52-54 |
| 7 | 0 | Cl | 4-F | H | $CH_3$ | H | H | H | H | H | H | 93 |
| 8 | 0 | $CF_3$ | H | H | $CH_3$ | H | H | H | H | H | H | 1502, 1450, 1329, 1220, 1191, 1170 |
| 9 | 0 | $C_2H_5$ | H | H | $CH_3$ | H | H | H | H | H | H | 1501, 1486, 1447, 1232, 1207, 1141 |

Tabelle 1: (Fortsetzung)

| Beispiel | n | $R^1$ | $R^2$ | $R^3$ | $R^4$ | $R^5$ | $R^6$ | $R^7$ | $R^8$ | $R^9$ | $R^{10}$ | Schmp. (°C) bzw. IR-Daten ($cm^{-1}$) |
|---|---|---|---|---|---|---|---|---|---|---|---|---|
| 10 | 0 | $OCH_3$ | H | H | $CH_3$ | H | H | H | H | H | H | 94 |
| 11 | 0 | H | H | H | H | H | $CH_3$ | H | H | H | H | 99-101 |
| 12 | 0 | F | H | H | H | H | $CH_3$ | H | H | H | H | 77-78 |
| 13 | 0 | Cl | H | H | $CH_3$ | H | $CH_3$ | H | H | H | H | 87 |
| 14 | 0 | Br | H | H | $CH_3$ | H | $CH_3$ | H | H | H | H | 81-82 |
| 15 | 0 | Cl | 4-F | H | $CH_3$ | H | $CH_3$ | H | H | H | H | 110-112 |
| 16 | 0 | $CF_3$ | H | H | $CH_3$ | H | $CH_3$ | H | H | H | H | 1501, 1450, 1328, 1217, 1189, 1165 |
| 17 | 0 | $C_2H_5$ | H | H | $CH_3$ | H | $CH_3$ | H | H | H | H | 1501, 1486, 1447, 1257, 1233, 1207 |
| 18 | 0 | $OCH_3$ | H | H | $CH_3$ | H | $CH_3$ | H | H | H | H | 1502, 1489, 1451, 1237, 1208, 1138 |
| 19 | 0 | F | H | H | $CH_3$ | H | H | H | H | H | $CH_3$ | 1502, 1485, 1271, 1241, 1205, 1121 |
| 20 | 0 | Cl | H | H | $CH_3$ | H | H | H | H | H | $CH_3$ | 1501, 1472, 1431, 1221, 1196, 1069 |
| 21 | 0 | Br | H | H | $CH_3$ | H | H | H | H | H | $CH_3$ | 1501, 1470, 1219, 1194, 1113, 895 |
| 22 | 0 | Cl | 4-F | H | $CH_3$ | H | H | H | H | H | $CH_3$ | 1503, 1489, 1241, 1206, 1116, 1051 |
| 23 | 0 | $CF_3$ | H | H | $CH_3$ | H | H | H | H | H | $CH_3$ | 1502, 1450, 1329, 1220, 1191, 1170 |
| 24 | 0 | $C_2H_5$ | H | H | $CH_3$ | H | H | H | H | H | $CH_3$ | 1501, 1486, 1447, 1257, 1233, 1207 |
| 25 | 0 | $OCH_3$ | H | H | $CH_3$ | H | H | H | H | H | $CH_3$ | 1501, 1489, 1451, 1240, 1208, 1138 |
| 26 | 0 | H | H | H | H | H | Cl | Cl | H | H | H | 117-119 |
| 27 | 0 | F | H | H | H | H | Cl | Cl | H | H | H | 105-109 |
| 28 | 0 | H | H | H | H | H | $CH_3$ | H | H | H | H | 99-101 |

Tabelle 1: (Fortsetzung)

| Beispiel | n | $R^1$ | $R^2$ | $R^3$ | $R^4$ | $R^5$ | $R^6$ | $R^7$ | $R^8$ | $R^9$ | $R^{10}$ | Schmp. (°C) bzw. IR-Daten ($cm^{-1}$) |
|---|---|---|---|---|---|---|---|---|---|---|---|---|
| 29 | 0 | F | H | H | H | H | $CH_3$ | H | H | H | H | 77-78 |
| 30 | 0 | H | H | H | H | H | Cl | Cl | $CH_3$ | $CH_3$ | H | 122-125 |
| 31 | 0 | F | H | H | H | H | Cl | Cl | $CH_3$ | $CH_3$ | H | 100-102 |
| 32 | 0 | H | H | H | $C_2H_5$ | H | H | H | H | H | H | |
| 33 | 0 | F | H | H | $C_2H_5$ | H | H | H | H | H | H | |
| 34 | 0 | H | H | H | $C_2H_5$ | H | $CH_3$ | H | H | H | H | |
| 35 | 0 | F | H | H | $C_2H_5$ | H | $CH_3$ | H | H | H | H | |
| 36 | 0 | H | H | H | $C_2H_5$ | H | H | H | H | H | $CH_3$ | |
| 37 | 0 | F | H | H | $C_2H_5$ | H | H | H | H | H | $CH_3$ | |
| 38 | 0 | H | H | H | $CH_3$ | $CH_3$ | H | H | H | H | H | |
| 39 | 0 | F | H | H | $CH_3$ | $CH_3$ | H | H | H | H | H | |
| 40 | 0 | H | H | H | $CH_3$ | $CH_3$ | $CH_3$ | H | H | H | H | |
| 41 | 0 | F | H | H | $CH_3$ | $CH_3$ | $CH_3$ | H | H | H | H | |
| 42 | 0 | H | H | H | $CH_3$ | $CH_3$ | H | H | H | H | $CH_3$ | |
| 43 | 0 | F | H | H | $CH_3$ | $CH_3$ | H | H | H | H | $CH_3$ | |

## Anwendungsbeispiele

In den folgenden Beispielen wurden die erfindungsgemäßen Verbindungen hinsichtlich ihrer Wirkung auf Schädlinge gegen die nachstehende Verbindung A des Standes der Technik verglichen:

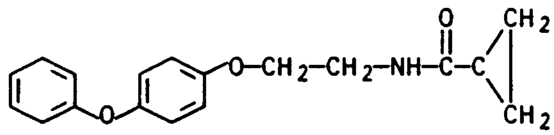

A

Die Reinheit der Substanzen wie der Vergleichsmittel lag bei > 95 %. Die Konzentrationen, bei denen die untersuchten Verbindungen eine 100 %ige bzw. 80 %ige Mortalität bzw. Hemmung bewirken, sind die jeweiligen Minimalkonzentrationen. (Wirkungsschwelle) Bei jeder Konzentration wurden mindestens zwei Versuche angesetzt und ein Mittelwert gebildet.

Beispiel A

Dysdercus intermedius (Baumwollwanze), ovizide Wirkung

Stecketiketten werden am oberen Rand mit Klebestreifenstücken (ca. 0,8 cm) beklebt. 24 Stunden vor Versuchsbeginn werden die Eier durch Eintauchen in das "Sammelgefäß" an dem Klebestreifenrand befestigt.

Die Eier werden für ca. 5 Sekunden in die wäßrige Wirkstoffaufbereitung getaucht, anschließend läßt man auf Filterpapier abtropfen. Dabei sollen die Eier nicht auf das Papier gelegt werden.

In diesem Versuch zeigte die Vergleichsverbindung A eine schwächere Wirkung als die Verbindungen der Beispiele 3, 11 und 12.

Beispiel B

Prodenia litura (ägypt. Baumwollwurm); Zuchtversuch

Die Zucht erfolgt in 100 ml Plastikbechern auf ca. 50 ml des Standard-Nährbodens, dem in flüssigen Zustand der Wirkstoff sorgfältig untergemischt wurde. Je Konzentration wird 1 Becher mit 5 Raupen des vierten Larvenstadiums angesetzt. Die Versuchstemperatur beträgt 25 bis 26 ° C. Die Beobachtung erstreckt sich bis zum Schlüpfen der Falter. Die Probe gilt als wirksam, wenn Riesenlarven produziert werden.

In diesem Versuch wies ein Vergleichsbeispiel A eine geringere Aktivität als die Verbindung des Beispiels Nr. 1 auf.

## Patentansprüche

**1.** Cyclopropancarboxamide der allgemeinen Formel I

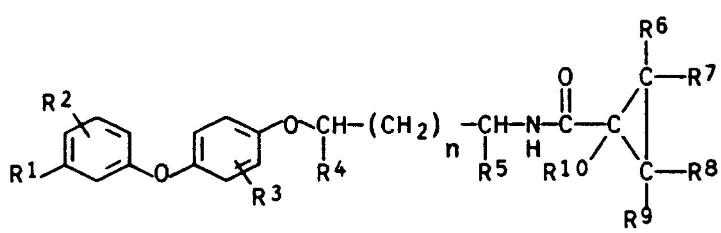

(I)

in der n für 0 oder 1 steht und die Substituenten folgende Bedeutung haben:

| | |
|---|---|
| $R^1$ | Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, Nitro oder Cyano |
| $R^2$ | Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, Nitro oder Cyano |
| $R^3$ | Wasserstoff, Halogen oder $C_1$-$C_3$-Alkyl |
| $R^4$, $R^5$ | Wasserstoff oder $C_1$-$C_4$-Alkyl und |
| $R^6$-$R^{10}$ | Wasserstoff, Halogen oder $C_1$-$C_4$-Alkyl |

mit der Maßgabe, daß die Reste $R^4$-$R^{10}$ nicht alle gleichzeitig für Wasserstoff stehen.

2. Verfahren zur Herstellung von Cyclopropancarboxamiden der allgemeinen Formel I gemäß Anspruch 1, dadurch gekennzeichnet, daß man ein 4-Phenoxyphenoxy-alkylamin der allgemeinen Formel II

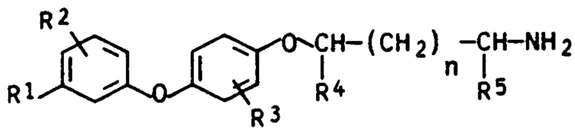

(II)

mit einem Cyclopropancarbonsäurehalogenid der allgemeinen Formel III

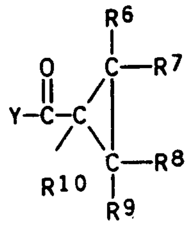

(III),

in der Y für Halogen steht, in Gegenwart eines Säureakzeptors umsetzt.

3. Schädlingsbekämpfungsmittel, enthaltend ein Cyclopropancarboxamid der Formel I gemäß Anspruch 1 neben hierfür üblichen Trägerstoffen.

4. Schädlingsbekämpfungsmittel nach Anspruch 3, dadurch gekennzeichnet, daß es 0, 1 bis 95 Gew.% eines Cyclopropancarboxamids der Formel I enthält.

5. Verfahren zur Bekämpfung von Schädlingen dadurch gekennzeichnet, daß man eine wirksame Menge eines Cyclopropancarboxamids der Formel I gemäß Anspruch 1 auf die Schädlinge bzw. deren Lebensraum einwirken läßt.

## Claims

1. A cyclopropanecarboxamide of the general formula I

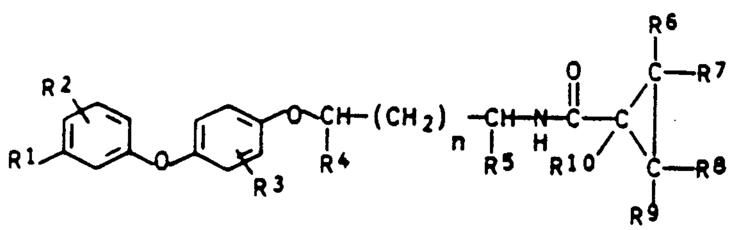

(I)

where n is 0 or 1,
$R^1$ and $R^2$ are each hydrogen, halogen, $C_1$-$C_4$-alkyl, $C_1$-$C_4$-haloalkyl, $C_1$-$C_4$-alkoxy, $C_1$-$C_4$-haloalkoxy, nitro or cyano,
$R^3$ is hydrogen, halogen or $C_1$-$C_3$-alkyl,
$R^4$ and $R^5$ are each hydrogen or $C_1$-$C_4$-alkyl and
$R^6$ to $R^{10}$ are each hydrogen, halogen or $C_1$-$C_4$-alkyl,
with the proviso that $R^4$ to $R^{10}$ are not all simultaneously hydrogen.

2. A process for the preparation of cyclopropanecarboxamides of the formula I as claimed in claim I, wherein a 4-phenoxyphenoxyalkylamine of the formula II

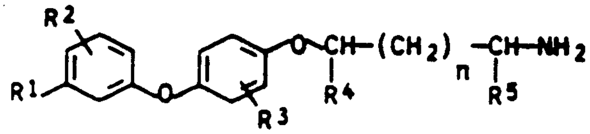

(II)

is reacted with a cyclopropanecarboxylic acid halide of the formula III

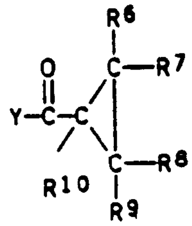

(III)

where Y is halogen, in the presence of an acid acceptor.

**3.** A pesticide containing a cyclopropanecarboxamide of the formula I as claimed in claim 1, and conventional carriers therefor.

**4.** A pesticide as claimed in claim 3, containing from 0.1 to 95% by weight of a cyclopropanecarboxamide of the formula I.

**5.** A method for controlling pests, wherein an effective amount of a cyclopropanecarboxamide of the formula I as claimed in claim 1 is allowed to act on the pests or their habitat.

**Revendications**

**1.** Cyclopropane-carboxamides de formule générale I

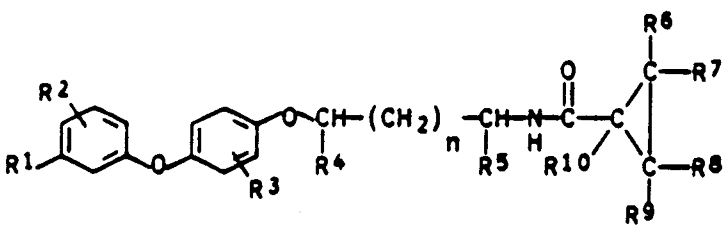

(I)

dans laquelle n est égal à 0 ou 1, et les symboles ont les significations suivantes :
$R^1$ représente l'hydrogène, un halogène, un groupe alkyle en C1 -C 4, halogénoalkyle en C 1-C 4, alcoxy en C 1-C 4, halogénoalcoxy en C 1-C 4, nitro ou cyano,
$R^2$ représente l'hydrogène, un halogène, un groupe alkyle en C 1-C 4, halogénoalkyle en C1-C 4, alcoxy en C 1-C 4, halogénoalcoxy en C 1-C 4, nitro ou cyano,
$R^3$ représente l'hydrogène, un halogène ou un groupe alkyle en C1-C 3,
$R^4$, $R^5$ représentent l'hydrogène ou un groupe alkyle en C 1-C 4, et
$R^6$ à $R^{10}$ représentent l'hydrogène, un halogène ou des groupes alkyle en C 1-C 4, ,sous réserve que les symboles $R^4$ à $R^{10}$ ne peuvent tous représenter simultanément l'hydrogène.

**2.** Procédé de préparation des cyclopropane-carboxamides de formule générale I de la revendication 1, caractérisé en ce que l'on fait réagir une 4-phénoxyphénoxy-alkylamine de formule générale II

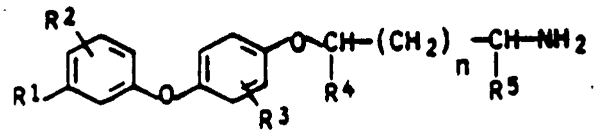

(II)

avec un halogénure d'acide cyclopropane-carboxylique de formule générale III

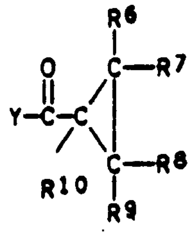

(III),

dans laquelle Y représente un halogène, en présence d'un accepteur d'acide.

3. Produit parasiticide contenant un cyclopropane-carboxamide de formule I de la revendication 1 avec des véhicules usuels à cet effet.

4. Produit parasiticide selon la revendication 3, caractérisé en ce qu'il contient de 0,1 à 95 % en poids d'un cyclopropane-carboxamide de formule I.

5. Procédé pour combattre les parasites, caractérisé en ce que l'on fait agir sur les parasites ou leur habitat une quantité efficace d'un cyclopropane-carboxamide de formule I de la revendication 1.